# EUROPÄISCHE PATENTANMELDUNG

(11) **EP 3 053 543 A1**
(43) Veröffentlichungstag der Anmeldung: **10.08.2016**
(21) Anmeldenummer: 15154319.6
(22) Anmeldetag: 09.02.2015
(51) Int. Cl.: A61F 2/00, A61N 1/375

(54) **VORRICHTUNG MIT OBERFLÄCHENSTRUKTUR ZUR EINBRINGUNG IN DEN MENSCHLICHEN ODER TIERISCHEN KÖRPER**

(71) Anmelder: BIOTRONIK SE & Co. KG, 12359 Berlin (DE)
(72) Erfinder: Dörr, Thomas, 12437 Berlin (DE); Kolberg, Gernot, 12161 Berlin (DE)
(74) Vertreter: Galander, Marcus

(57) **Zusammenfassung**

Die Erfindung betrifft eine Vorrichtung (110) zur Einbringung in einen menschlichen oder tierischen Körper, wobei die Vorrichtung eine Oberfläche (610) mit mindestens partieller Oberflächenstruktur (140, 140') aufweist, wobei der Haftreibungskoeffizient zwischen der Oberflächenstruktur (140, 140') und einer körperinternen Grenzfläche anisotrop ist.

## Beschreibung

Die vorliegende Erfindung betrifft eine Vorrichtung zur Einbringung in den menschlichen oder tierischen Körper, beispielsweise ein medizinisches Implantat. Insbesondere betrifft die Erfindung die Einbringung von Implantaten an schwer zugänglichen Implantationsorten.

Die Einbringung von Implantaten in den menschlichen oder tierischen Körper findet typischerweise durch einen chirurgischen Eingriff statt. Dabei weist der Eingriff in Abhängigkeit des Implantationsorts, der Art des Implantats, der Implantationsmethodik und der Implantationswerkzeuge einen verschiedenartigen Umfang der operativen Invasivität für den Patienten sowie eine unterschiedliche Operationsdauer und Schwierigkeitsstufe für den Operateur auf.

Bekannt im Stand der Technik sind Implantate mit Oberflächenstrukturen und - Beschichtungen, die dazu dienen, die Schnittstelle zwischen Implantat und Gewebe chemisch, physikalisch und biologisch derart zu modifizieren, dass die Verträglichkeit zum biologischen Milieu verbessert wird. So sind Gefäßstents mit Beschichtungen bekannt, welche antithrombogen wirkende Medikamente enthalten. Bekannt sind des Weiteren Knochenimplantate, die mit einer bioaktiven Oberflächenstruktur versehen sind, sodass natürlicher Knochen auf schnelle Art und Weise fest mit dem Implantat verwächst. Antimikrobielle Oberflächenbeschichtungen verringern das Infektionsrisiko und ermöglichen eine verbesserte Biokompatibilität des Implantats.

Weiterhin sind Implantate bekannt, die über an der Implantatsoberfläche angebrachte Mittel verfügen, welche eine Verschiebung oder ein Verrutschen des Implantats am Implantationsort verhindern und somit eine feste Position des Implantats sichern.

US 2012/0220917 A1 offenbart ein okulares Implantat mit fingerartigen Einheiten, die am Implantationsort expandieren und somit das Implantat am Implantationsort fixieren.

US 2014/0180065 A1 offenbart ein röntgenopakes Implantat, welches über Spitzen auf dem Implantatskörper verfügt, die das Implantat am Implantationsort verankern.

Das gesundheitliche Risiko für den Patienten sowie die direkten und indirekten Kosten einer Implantation erhöhen sich bei steigender operativer Invasivität, Operationsdauer und Schwierigkeitsstufe eines Eingriffs. Diese Risikofaktoren sind besonders hoch, wenn sich der Implantationsort an einer schwer zugänglichen Stelle des Körpers befindet. Wünschenswert wären daher Mittel zur Vereinfachung der Einbringung von Implantaten an schwer zugänglichen Implantationsorten.

Es ist Aufgabe der vorliegenden Erfindung, eine verbesserte Vorrichtung für die Einbringung von Implantaten an schwer zugänglichen oder bisher unzugänglichen Implantationsorten des Körpers zu schaffen.

Die Aufgabe der vorliegenden Erfindung wird durch eine Vorrichtung zur Einbringung in einen menschlichen oder tierischen Körper gelöst, wobei die Vorrichtung eine Oberfläche mit mindestens partieller Oberflächenstruktur aufweist und die Oberflächenstruktur dadurch gekennzeichnet ist, dass der Haftreibungskoeffizient zwischen der Oberflächenstruktur und einer körperinternen Grenzfläche anisotrop ist. Dabei kann die erfindungsgemäße Vorrichtung als ein Implantat oder ein Beförderungsmittel für ein Implantat ausgeführt sein.

Ein beispielhafter schwer zugänglicher Implantationsort wäre ein Gebiet, an dem verschiedene körperinterne Grenzflächen, wie z. B. Gewebsoberflächen eng bei- und/oder aneinander liegen. Die vorliegende Erfindung ermöglicht die Einbringung und Beförderung einer Vorrichtung zwischen zwei eng aneinander liegenden Gewebsoberflächen, da die Oberflächenstruktur eine gerichtete Verschiebung der Vorrichtung zwischen diesen Gewebsoberflächen erlaubt. So wird die Vorrichtung an den gewünschten Implantationsort gebracht, wenn eine relative Bewegung der Gewebsoberflächen zueinander stattfindet. Eine solche relative Bewegung kann entweder durch körpereigene Bewegungen der Gewebsoberflächen stattfinden oder von außen induziert werden. Die Oberflächenstruktur kann fester Bestandteil eines Implantats sein. Alternativ kann die Oberflächenstruktur auch Teil einer Trägereinrichtung sein, die zur Beförderung eines Implantats ausgerichtet ist und gestaltet ist, dass das Implantat am Implantationsort von der Trägereinrichtung getrennt werden kann.

In einer bevorzugten Ausführungsform der Erfindung ist der Haftreibungskoeffizient zwischen der Oberflächenstruktur und der körperinternen Grenzfläche minimal in einer ersten Verschiebungsrichtung und maximal in einer zweiten Verschiebungsrichtung, wobei die erste Verschiebungsrichtung der zweiten Verschiebungsrichtung entgegengesetzt sein kann.

Die erfindungsgemäße Oberflächenstruktur ist dazu ausgelegt, eine Verschiebung der Vorrichtung in eine kontrollierte Verschiebungsrichtung zu ermöglichen.

In einer Ausführungsform der Erfindung ist die Anisotropie des Haftreibungskoeffizienten zwischen der Oberflächenstruktur und der körperinternen Grenzfläche aufhebbar.

Auch kann die Anisotropie des Haftreibungskoeffizienten durch eine materialtechnische Veränderung der Oberflächenstruktur aufgehoben werden, die mechanischer, thermischer, elektrischer oder chemischer Art sein kann.

Die Aufhebung der Anisotropie des Haftreibungskoeffizienten kann auch durch einen Auslöser eingeleitet werden. Dabei kann der Auslöser mechanischer, thermischer, elektrischer oder chemischer Natur sein.

Alternativ kann, um die Anisotropie des Haftreibungskoeffizienten aufzuheben, die Oberflächenstruktur nach Erreichen der Vorrichtung am gewünschten Implantationsort von einer zusätzlichen Hüllsubstanz oder -Struktur abgedeckt werden.

In einer weiteren Ausführungsform der Erfindung ist die Oberflächenstruktur von der Vorrichtung trennbar.

Beispielsweise wird dafür die Oberflächenstruktur mittels einer lösbaren Fixierung auf der Vorrichtung befestigt. Die Fixierung kann z.B. mechanisch lösbar sein, wie durch einen Faden oder andere mechanische Vorrichtungen. Auch kann die Vorrichtung eine Folie auf der Oberflächenstruktur aufweisen, wobei die Folie von der Vorrichtung ablösbar ist.

In einer weiteren Ausführungsform der Erfindung besteht die Oberflächenstruktur aus einem resorbierbaren Material.

In einer möglichen Ausführungsform kann die Resorption durch einen Auslöser mechanischer, thermischer, elektrischer oder chemischer Art eingeleitet werden.

In einer weiteren Ausführungsform weist die erfindungsgemäße Vorrichtung ein Fixiermaterial auf, über das die Vorrichtung mit der körperinternen Grenzfläche dauerhaft verbindbar ist.

Beispiele für ein solches Fixiermaterial sind Gewebekleber, Federelemente, Fixierhaken etc. In einer Ausführungsform wird das Fixiermaterial aktiviert, sodass es die Vorrichtung mit der körperinternen Grenzfläche verbindet, nachdem die Oberflächenstruktur von der Vorrichtung getrennt wird oder nachdem die Oberflächenstruktur resorbiert wurde.

Alternativ kann die Aufhebung der Anisotropie des Haftreibungskoeffizienten durch ein die Anisotropie des Haftreibungskoeffizienten aufhebbares Material stattfinden, welches auf der Oberflächenstruktur aufbringbar ist.

Auch kann das auf der Oberflächenstruktur aufbringbare Material zusätzlich dazu ausgelegt sein, die Oberflächenstruktur mit der körperinternen Grenzfläche dauerhaft zu verbinden.

In einer bevorzugten Ausführungsform weist die Oberflächenstruktur Elemente auf, die schräg zur Oberfläche orientiert sind. Dabei können die Elemente in Form von Fasern, Nadeln, Noppen, Lamellen, Spitzen, Zacken oder Stiften ausgeführt sein.

Im Sinne der Erfindung ist eine schräge Orientierung der Elemente zur Oberfläche weitestgehend derart zu verstehen, dass der Winkel zwischen der Ausrichtung jedes Elements zur Oberfläche der Vorrichtung kleiner als 90° beträgt.

Geeignete Materialien, aus denen die Elemente der Oberflächenstruktur gefertigt sein können, wären unter anderem Titan, Edelstahl, Silikon, biokompatible Polymere, bioresorbierbare Polymere wie Polymere der Glykolsäure oder Copolymere aus Glykolyd und Lactid oder Polymere aus Dioxanon, Polyetheretherketon (PEEK), bioresorbierbare Magnesiumverbindungen, resorbierbare Zuckerstrukturen wie Mannitol.

In einer weiteren Ausführungsform sind an der Vorrichtung Mittel zur Steuerung einer Verschiebungsrichtung der Vorrichtung angebracht.

Die Mittel zur Steuerung der Verschiebungsrichtung können dazu dienen, die die Verschiebungsrichtung und/oder -Strecke während der Einbringung der Vorrichtung in den Körper von außen kontrollieren und ändern zu können. Derartige Mittel können beispielsweise in Form von Steuerfäden oder von Mandrins ausgeführt sein.
- Fig. 1: Ausführungsform der erfindungsgemäßen Vorrichtung;
- Fig. 2: Ausführungsform der erfindungsgemäßen Vorrichtung mit an der Vorrichtung angebrachten Mitteln zur Steuerung der Verschiebungsrichtung;
- Fig. 3a: Ausführungsform der erfindungsgemäßen Vorrichtung als Trägereinrichtung, bei der die Oberflächenstruktur Teil der Trägereinrichtung ist;
- Fig. 3b: Am Implantationsort kann das Implantat von der Trägereinrichtung getrennt werden;
- Fig. 4a: Ausführungsform der erfindungsgemäßen Vorrichtung, bei der die Vorrichtung eine Folie mit der Oberflächenstruktur aufweist;
- Fig. 4b: Folie mit Oberflächenstruktur ist von der Vorrichtung ablösbar;
- Fig. 5a: Ausführungsform der erfindungsgemäßen Vorrichtung, bei der die Anisotropie des Haftreibungskoeffizienten der Oberflächenstruktur aufhebbar ist;
- Fig. 5b: Erfindungsgemäße Vorrichtung nach Aufhebung der Anisotropie des Haftreibungskoeffizienten der Oberflächenstruktur
- Fig. 6a-e: Mögliche Ausführungsformen der Elemente der Oberflächenstruktur
- Fig. 7a-c: Mögliche Ausführungsformen der Elemente der Oberflächenstruktur als Einzelelemente oder Lamellenstruktur
- Fig. 8a-b: Winkel zwischen Ausrichtung eines Elements zur Oberfläche der Vorrichtung
- Fig. 9: Detaillierte Bestimmungsmethode der Definition einer schrägen Orientierung eines Elements der Oberflächenstruktur zur Oberfläche der Vorrichtung

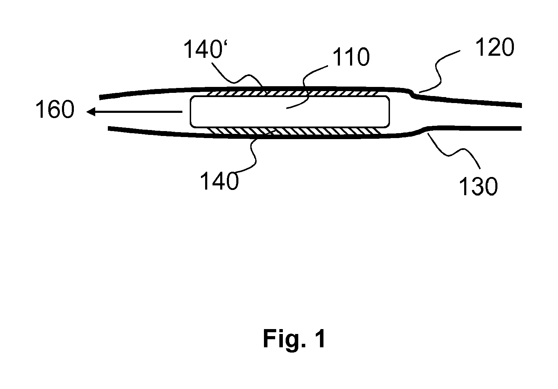

Fig. 1 zeigt eine schematische Darstellung einer möglichen Ausführungsform der vorliegenden Erfindung. Abgebildet ist Vorrichtung 110, die eine erfindungsgemäße Oberflächenstruktur 140 und 140' aufweist und sich zwischen zwei Gewebsoberflächen 120 und 130 befindet. Durch die Anisotropie der Haftreibungskoeffizienten der Oberflächenstruktur 140 und 140' besitzt die Vorrichtung eine gerichtete Verschiebungsrichtung 160. Als mögliche Ausführungsform stellt 110 einen epikardialen Herzschrittmacher dar, 120 und 130 repräsentieren das Peri- bzw. Epikard.

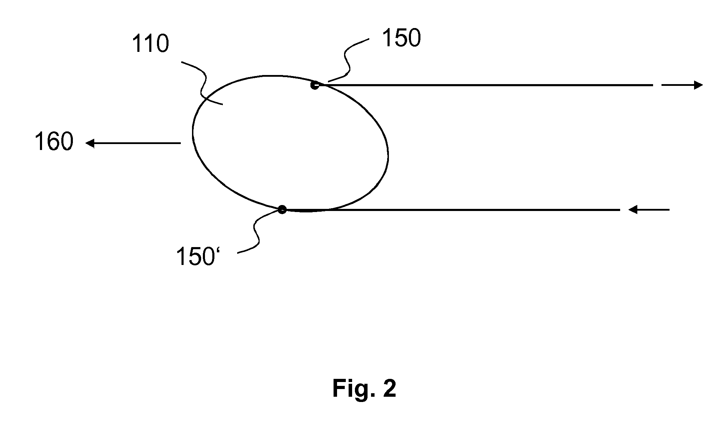

Fig. 2 zeigt eine mögliche Ausführungsform der Erfindung, wobei an den Seiten der Vorrichtung 110 Mittel 150 und 150'zur Steuerung der Verschiebungsrichtung 160 angebracht sind. In einer Ausführungsform können die Mittel zur Steuerungsrichtung als Steuerfäden oder in Form von dünnen, leicht verformten und flexiblen Mandrins ausgeführt sein. Ist Vorrichtung 110 ein epikardialer Herzschrittmacher, so kann dieser zunächst zwischen Perikard und Epikard geschoben werden, um anschließend unter Ausnutzung der Eigenbewegung des Herzens bzw. relativen Bewegung von Perikard und Epikard zueinander und durch Betätigung der Mittel zur Steuerung der Verschiebungsrichtung 150 und 150' an den gewünschten Implantationsort gebracht zu werden.

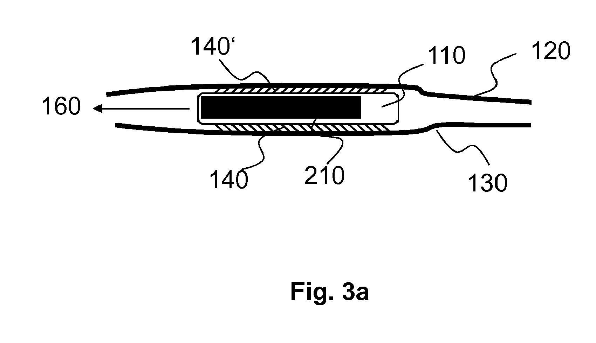

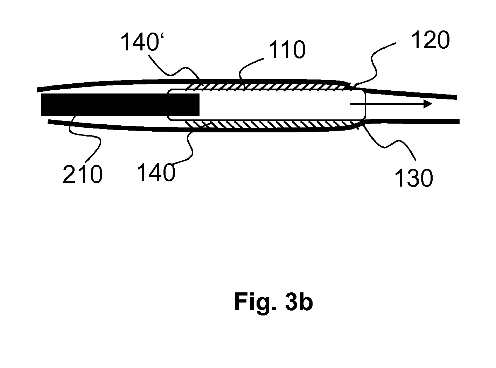

Fig. 3a und 3b zeigen eine Ausführungsform der Erfindung, bei welcher Vorrichtung 110 als Trägereinrichtung gestaltet ist, welche die erfindungsgemäße Oberflächenstruktur 140 und 140' aufweist. Die Vorrichtung 110 befindet sich zwischen zwei Gewebsoberflächen 120 und 130, besitzt eine gerichtete Verschiebungsrichtung 160 und ist dazu ausgelegt, ein Implantat 210 zu transportieren. Dafür ist das Implantat 210 in oder an der Vorrichtung 110 fixiert, beispielsweise durch einen als Schlaufe geführten Faden. Nach Erreichen des Implantationsortes kann das Implantat 210 von der Vorrichtung 110 getrennt werden, in dem die Fixierung gelöst wird. Alternativ kann die Fixierung auch derart gestaltet sein, dass das Implantat 210 vor einer vollständigen Lösung der Fixierung wieder mit der Vorrichtung 110 verbunden werden kann, sodass eine Korrektur der Position des Implantats 210 möglich ist.

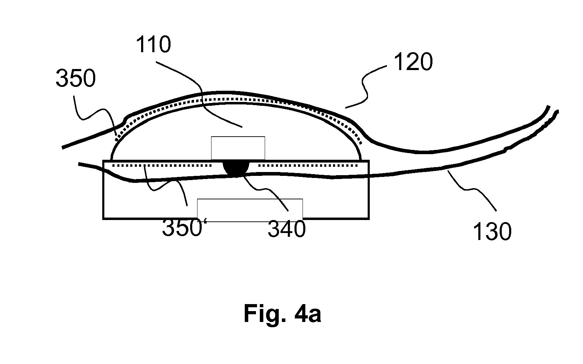

In Fig. 4a ist eine weitere Ausführungsform der Erfindung gezeigt. Dabei ist Vorrichtung 110 als epikardialer Herzschrittmacher ausgeführt, welcher sich zwischen zwei Gewebsoberflächen 120 und 130 befindet, beispielsweise zwischen Epikard und Perikard.

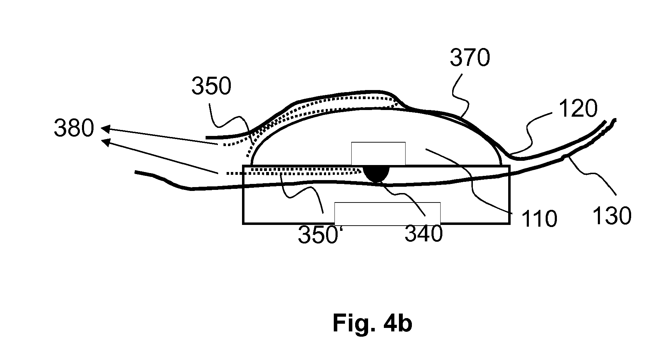

Die Vorrichtung besitzt in einer Ausführungsform einen Stimulationspol 340. Die Oberflächenstruktur ist auf den Folien 350 und 350' aufgebracht, welche auf der Vorrichtung 110 fixiert sind. Nach Erreichen des Implantationsortes können die Folien 350 und 350' von der Vorrichtung 110 getrennt werden, gezeigt in Fig. 4b, beispielsweise durch Abziehen in Richtung 380, wobei zusätzlich ein Mittel 370, wie z.B. ein Gewebekleber, zur Fixierung der Vorrichtung 110 mit den Gewebsoberflächen 120 und 130 aktiviert werden kann.

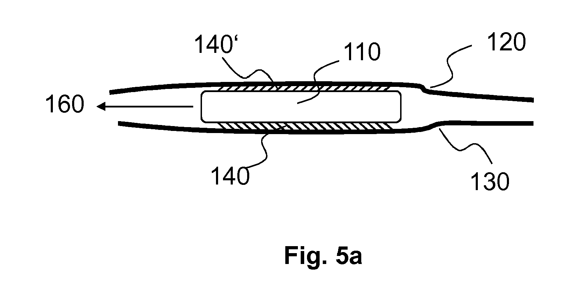

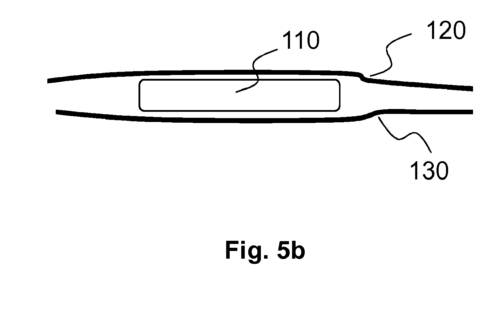

Fig. 5a und 5b zeigen eine weitere Ausführungsform der Erfindung, bei der die Anisotropie des Haftreibungskoeffizienten der Oberflächenstruktur 140 und 140' aufhebbar ist. Gezeigt ist Vorrichtung 110, welche sich zwischen zwei Gewebsoberflächen 120 und 130 befindet und eine gerichtete Verschiebungsrichtung 160 aufweist. Die Aufhebung der Anisotropie des Haftreibungskoeffizienten der Oberflächenstruktur 140 und 140' kann beispielsweise, nachdem die Vorrichtung 110 an den gewünschten Implantationsort gebracht wurde, durch einen Auslöser mechanischer, thermischer, elektrischer oder chemischer Art initiiert werden. Alternativ kann die Oberflächenstruktur auch aus einem resorbierbaren Material bestehen, welches nach einer vorzugsweise kurzen Zeitspanne vom Gewebe aufgenommen wird.

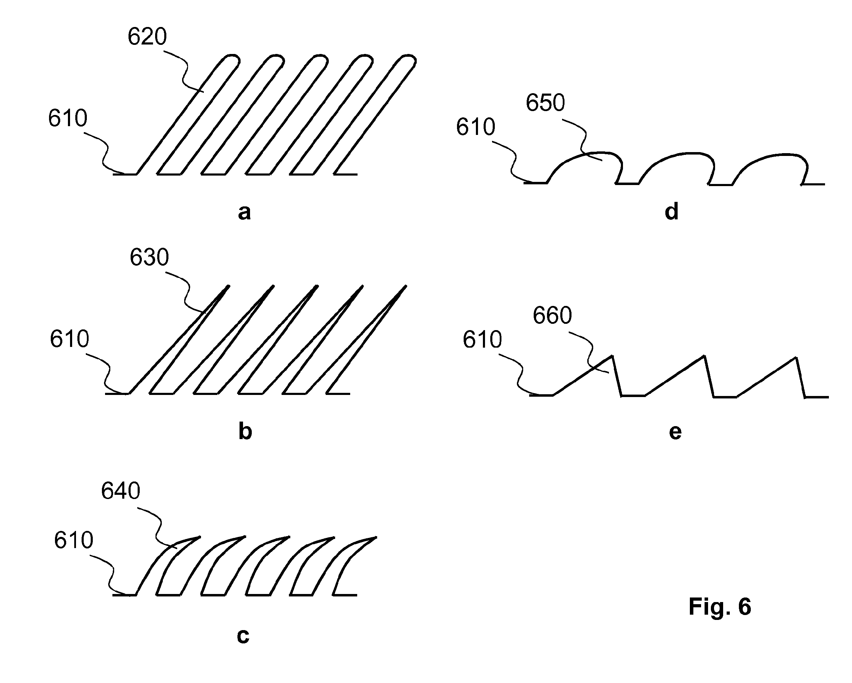

Fig. 6a bis 6e illustrieren beispielhafte Ausführungsformen der Elemente der Oberflächenstruktur. So können diese als Stifte 620, Nadeln 630, gebogene/flexible Spitzen bzw. Fasern 640, Noppen 650 oder Zacken 660 ausgebildet sein, die schräg zur Oberfläche der Vorrichtung 610 orientiert sind.

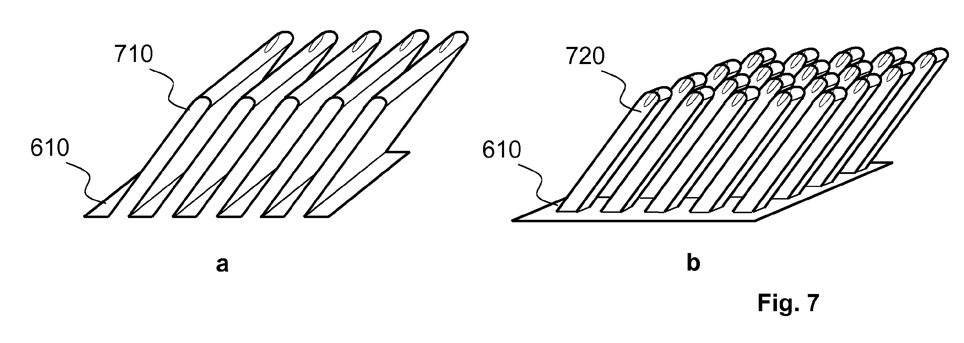

In Fig. 7a und 7b sind beispielhafte räumliche Ausbildungsformen für die Elemente der Oberflächenstruktur auf der Oberfläche der Vorrichtung illustriert. Dabei sind die zur Oberfläche der Vorrichtung 610 schräg orientierten Elemente in Fig. 7a als Lamellen 710 und in Fig. 7b als einzelne Elemente 720 ausgeführt.

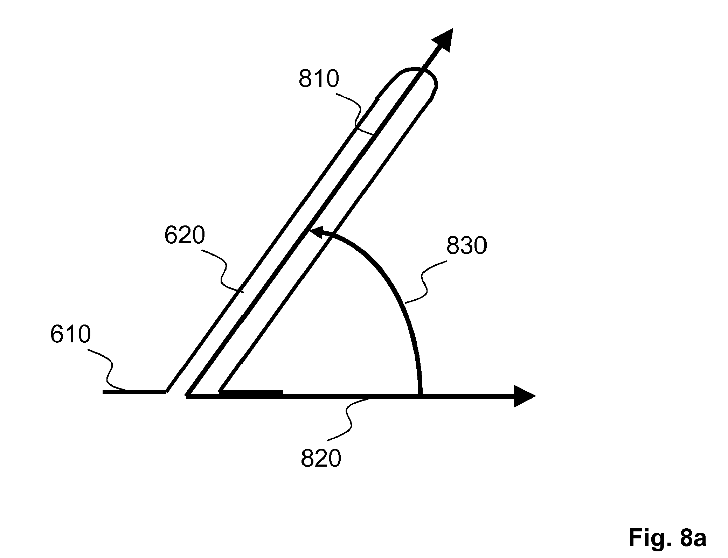

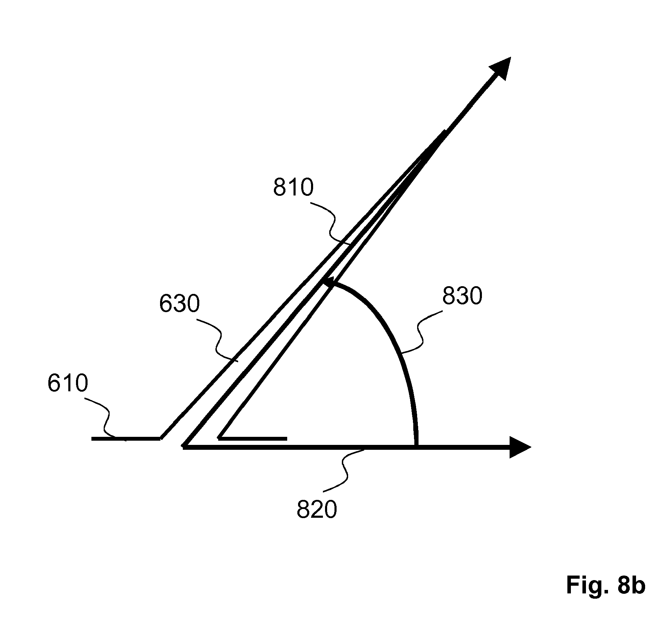

Fig. 8a veranschaulicht die schräge Orientierung eines Elements der Oberflächenstruktur, hier in der Form des Stiftes 620, zur Oberfläche der Vorrichtung 610. Im Sinne der Erfindung ist eine solche schräge Orientierung gegeben, wenn ein Winkel 830, dessen Schenkel gebildet werden aus einem Vektor der Ausrichtung eines Elements 810 und einem Vektor 820, der im Ansatz des Elements 840 seinen Startpunkt besitzt und parallel bzw. tangential zur Oberfläche der Vorrichtung 610 verläuft, kleiner als 90° beträgt. Fig. 8b veranschaulicht die schräge Orientierung eines Elements in Form einer Nadel 630 zur Oberfläche der Vorrichtung 610.

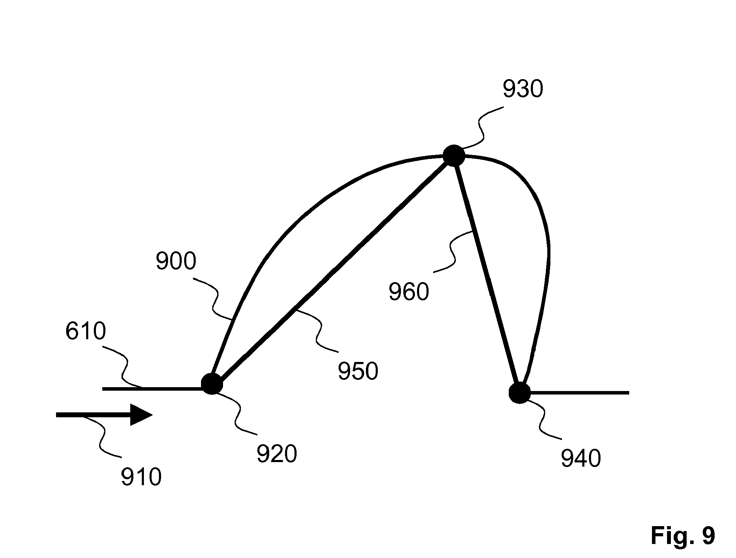

Tritt der Fall ein, dass ein Winkel bzw. sind die zwei den winkelbildenden Schenkel nicht eindeutig erkennbar oder bestimmbar sind, beispielsweise aufgrund einer speziellen Geometrie der Elemente der Oberflächenstruktur, so ist für die Definition der schrägen Orientierung der Elemente zur Oberfläche der folgenden Methode, die in Fig. 9 beispielhaft für die Ausführungsform eines Elements als Noppe illustriert ist, vorzuziehen. Es wird ein Querschnitt durch ein Element gebildet, wobei die Querschnittsebene aufgespannt wird von
- der Bewegungsrichtung mit dem kleinsten Haftreibungskoeffizienten der Oberflächenstruktur und
- dem Normalvektor der Oberfläche der Vorrichtung.

Dabei wird ein Querschnitt generiert, wobei 900 die Kante darstellt, welche das Element umreißt. Anschließend wird im Querschnitt die Kante eines Elements in eine Richtung 910 abgefahren. Dabei werden folgende Punkte in der angegebenen Reihenfolge bestimmt:
- Punkt 920 auf der Kante des Elements, der eine kleinste Distanz zur Oberfläche der Vorrichtung besitzt
- Punkt 930 auf der Kante des Elements, der den ersten Punkt mit der größten Distanz zur Oberfläche der Vorrichtung darstellt, der hinter Punkt 920 liegt
- Punkt 940 auf der Kante des Elements, der hinter A liegt und die kleinste Distanz zur Oberfläche der Vorrichtung besitzt.

Es werden zwei Linien 950 und 960 gebildet, wobei
- Linie 950 die Verbindung zwischen Punkt 920 und 930 darstellt
- Linie 960 die Verbindung zwischen Punkt 930 und 940 darstellt.

So wird die Orientierung eines Elements zur Oberfläche als schräg bezeichnet, wenn Linie 950 und Linie 960 nicht gleich lang sind.

Eine bevorzugte Anwendungsmöglichkeit für die vorliegende Erfindung stellt ein Herzschrittmacher dar, der am Epikard (äußerste Schicht der Herzwand) implantiert wird, im Folgenden als epikardialer Herzschrittmacher bezeichnet. Das Epikard liegt eng am Perikard (Herzbeutel) an, wobei sich die zwei Gewebsoberflächen durch die natürliche Pumpbewegung des Herzens andauernd relativ zueinander bewegen. Unter Anwendung von Implantationsmethoden nach dem Stand der Technik wäre somit das Epikard als Implantationsort schwer zugänglich. Mit einem mit der erfindungsgemäßen Oberflächenstruktur versehenen Implantat ist es möglich, das Implantat an einen vorgesehenen Implantationsort am Epikard einzubringen, in dem die Eigenbewegung des Herzens und die relative Bewegung von Epikard und Perikard zueinander genutzt werden. Dazu wird das Implantat zuerst an eine geeignete Stelle zwischen Epikard und Perikard platziert, sodass die Oberflächenstruktur in Kontakt zu mindestens einer der Gewebsschichten steht. Durch die Anisotropie des Haftreibungskoeffizienten der Oberflächenstruktur und der relativen Bewegung beider Gewebsschichten zueinander wird so das Implantat entlang der zwei Grenzschichten in eine Richtung bewegt, die bezüglich der Oberflächenstruktur und deren erfindungsgemäßen Eigenschaften eine geringe Haftreibung aufweist.

Die vorliegende Erfindung ist anwendbar auf verschiedenartige aktive und passive, temporäre und dauerhafte Implantate, wie zum Beispiel kardiale Stimulatoren, Neurostimulatoren, Pulsgeneratoren, alle Arten von Sensoren und Sensorleitungen, Medikamententräger, Radionuklidträger, medizinische Marker wie z.B. Röntgenmarker, temporäre und permanente Katheter, Naht- und Verschlusssysteme.

Beispielhafte weitere Implantationsorte stellen sub- oder intrameningiale, intrapleurale Lagen sowie Positionen zwischen Organen oder Organspalten (z.B. Leber, Niere, Magen, Zwerchfell), Harnröhre, Blutgefäßen dar. Dabei muss für die Einbringung der Vorrichtung nicht zwingend eine körpereigene Bewegung einer körperinternen Grenzfläche vorhanden sein bzw. genutzt werden. Eine solche Bewegung kann auch von außen induziert werden.

## Patentansprüche

1. Vorrichtung (110) zur Einbringung in einen menschlichen oder tierischen Körper, wobei die Vorrichtung eine Oberfläche (610) mit mindestens partieller Oberflächenstruktur (140, 140') aufweist, wobei der Haftreibungskoeffizient zwischen der Oberflächenstruktur (140, 140') und einer körperinternen Grenzfläche anisotrop ist.

2. Vorrichtung (110) nach Anspruch 1, wobei die Vorrichtung ein Implantat oder ein Beförderungsmittel für ein Implantat ist.

3. Vorrichtung (110) nach Anspruch 1 oder 2, wobei der Haftreibungskoeffizient zwischen der Oberflächenstruktur (140, 140') und der körperinternen Grenzfläche minimal in einer ersten Verschiebungsrichtung (160) und maximal in einer zweiten Verschiebungsrichtung ist.

4. Vorrichtung (110) nach Anspruch 3, wobei die erste Verschiebungsrichtung (160) der zweiten Verschiebungsrichtung entgegengesetzt ist.

5. Vorrichtung (110) nach einem der vorhergehenden Ansprüche, wobei die Anisotropie des Haftreibungskoeffizienten aufhebbar ist.

6. Vorrichtung (110) nach einem der vorhergehenden Ansprüche, wobei die Oberflächenstruktur (140, 140') von der Vorrichtung (110) trennbar ist.

7. Vorrichtung (110) nach einem der vorhergehenden Ansprüche, wobei die Oberflächenstruktur (140, 140') aus einem resorbierbaren Material besteht.

8. Vorrichtung (110) nach einem der vorhergehenden Ansprüche, wobei die Vorrichtung ein Fixiermaterial (370) aufweist, über das die Vorrichtung (110) mit der körperinternen Grenzfläche dauerhaft verbindbar ist.

9. Vorrichtung (110) nach einem der Ansprüche 1 bis 5, wobei ein die Anisotropie des Haftreibungskoeffizienten aufhebbares Material auf der Oberflächenstruktur (140, 140') aufbringbar ist.

10. Vorrichtung (110) nach Anspruch 9, wobei die Oberflächenstruktur (140, 140') über das die Anisotropie des Haftreibungskoeffizienten aufhebbare Material mit der körperinternen Grenzfläche dauerhaft verbindbar ist.

11. Vorrichtung (110) nach einem der vorhergehenden Ansprüche, wobei die Oberflächenstruktur (140, 140') Elemente (620, 630, 640, 650, 660, 710, 720) aufweist, die schräg zur Oberfläche orientiert sind.

12. Vorrichtung (110) nach Anspruch 11, wobei die Elemente (620, 630, 640, 650, 660, 710, 720) in Form von Fasern, Nadeln, Noppen, Lamellen, Spitzen, Zacken oder Stiften ausgeführt sind.

13. Vorrichtung (110) nach einem der vorhergehenden Ansprüche, wobei an der Vorrichtung (110) Mittel zur Steuerung einer Verschiebungsrichtung (150, 150') der Vorrichtung (110) angebracht sind.
